# EUROPEAN PATENT APPLICATION

(11) **EP 2 096 168 A1**
(43) Date of publication of application: **02.09.2009**
(21) Application number: 07849806.0
(22) Date of filing: 07.12.2007
(51) Int. Cl.: C12N 15/09, G01N 33/574, C07K 14/46, C07K 16/18

(54) **DIAGNOSTIC AGENT FOR MESOTHELIOMA, DIAGNOSIS KIT FOR MESOTHELIOMA, AND DIAGNOSIS METHOD FOR MESOTHELIOMA**

(30) Priority: 08.12.2006 JP 2006331409
(71) Applicant: Immuno-Biological Laboratories Co., Ltd., Takasaki-shi Gunma 370-0831 (JP)
(72) Inventor: HINO, Okio, Higashikurume-shi Tokyo 203-0012 (JP); SEITO, Tsutomu, Takasaki-shi Gunma 370-0831 (JP)
(74) Representative: Woods, Geoffrey Corlett
(86) International application number: PCT/JP2007/001373
(87) International publication number: WO 2008/068906

(57) **Abstract**

The present invention provides a diagnostic agent and the like for mesothelioma which is able to effectively distinguish mesothelioma patients from healthy volunteers, screen mesothelioma patients from among patients with a history of exposure to asbestos, and distinguish mesothelioma patients from patients with other diseases, such as lung cancer, at a higher sensitivity than hitherto reported mesothelioma diagnostic kits or the like. The invention relates to a diagnostic agent for mesothelioma, including, as the active ingredient, an antibody that recognizes an N-terminal side fragment from among fragments formed by leaving 31 kDa secreted Erc/MPF/mesothelin in blood, or in serum that has been separated from blood, at room temperature for at least three hours.

SEQUENCE LISTING

<110>  Immuno-Biological Laboratories Co., Ltd.

<120>  Diagnostic reagent, diagnostic kit and diagnostic method for
mesothelioma

<130>  IB07002PWO

<160>  9

<170>  PatentIn version 3.1

<210>  1
<211>  295
<212>  PRT
<213>  Homo sapiens

<400>  1

Met Ala Leu Pro Thr Ala Arg Pro Leu Leu Gly Ser Cys Gly Thr Pro
1               5                   10                  15

Ala Leu Gly Ser Leu Leu Phe Leu Leu Phe Ser Leu Gly Trp Val Gln
            20                  25                  30

Pro Ser Arg Thr Leu Ala Gly Glu Thr Gly Gln Glu Ala Ala Pro Leu
        35                  40                  45

Asp Gly Val Leu Ala Asn Pro Pro Asn Ile Ser Ser Leu Ser Pro Arg
    50                  55                  60

Gln Leu Leu Gly Phe Pro Cys Ala Glu Val Ser Gly Leu Ser Thr Glu
65                  70                  75                  80

Arg Val Arg Glu Leu Ala Val Ala Leu Ala Gln Lys Asn Val Lys Leu
                85                  90                  95

Ser Thr Glu Gln Leu Arg Cys Leu Ala His Arg Leu Ser Glu Pro Pro
            100                 105                 110

Glu Asp Leu Asp Ala Leu Pro Leu Asp Leu Leu Leu Phe Leu Asn Pro
        115                 120                 125

Asp Ala Phe Ser Gly Pro Gln Ala Cys Thr His Phe Phe Ser Arg Ile
    130                 135                 140

Thr Lys Ala Asn Val Asp Leu Leu Pro Arg Gly Ala Pro Glu Arg Gln
145                 150                 155                 160

Arg Leu Leu Pro Ala Ala Leu Ala Cys Trp Gly Val Arg Gly Ser Leu
                165                 170                 175

Leu Ser Glu Ala Asp Val Arg Ala Leu Gly Gly Leu Ala Cys Asp Leu
            180                 185                 190

Pro Gly Arg Phe Val Ala Glu Ser Ala Glu Val Leu Leu Pro Arg Leu
        195                 200                 205

Val Ser Cys Pro Gly Pro Leu Asp Gln Asp Gln Gln Glu Ala Ala Arg
    210                 215                 220

Ala Ala Leu Gln Gly Gly Gly Pro Pro Tyr Gly Pro Pro Ser Thr Trp
225                 230                 235                 240

Ser Val Ser Thr Met Asp Ala Leu Arg Gly Leu Leu Pro Val Leu Gly
                245                 250                 255

Gln Pro Ile Ile Arg Ser Ile Pro Gln Gly Ile Val Ala Ala Trp Arg
            260                 265                 270

Gln Arg Ser Ser Arg Asp Pro Ser Trp Arg Gln Pro Glu Arg Thr Ile
        275                 280                 285

Leu Arg Pro Arg Phe Arg Arg
    290                 295

<210>  2
<211>  18
<212>  PRT
<213>  Artificial

<220>
<223>  peptide(a)

<400>  2

Ser Arg Thr Leu Ala Gly Glu Thr Gly Gln Glu Ala Ala Pro Leu Asp
1               5                   10                  15

Gly Val

<210>  3
<211>  6
<212>  PRT
<213>  Artificial

<220>
<223>  peptide(b)

<400>  3

Gly Phe Pro Cys Ala Glu
1               5


<210>  4
<211>  6
<212>  PRT
<213>  Artificial

<220>
<223>  peptide(c)

<400>  4

Pro Gln Ala Cys Thr His
1               5


<210>  5
<211>  19
<212>  PRT
<213>  Artificial

<220>
<223>  peptide(d)

<400>  5

Cys Pro Gly Pro Leu Asp Gln Asp Gln Gln Glu Ala Ala Arg Ala Ala
1               5                   10                  15


Leu Gln Gly



<210>  6
<211>  885
<212>  DNA
<213>  Homo sapiens

<400>  6
atggccttgc caacggctcg acccctgttg gggtcctgtg ggacccccgc cctcggcagc      60

ctcctgttcc tgctcttcag cctcggatgg gtgcagccct cgaggaccct ggctggagag 120

acagggcagg aggctgcgcc cctggacgga gtcctggcca acccacctaa catttccagc 180

ctctcccctc gccaactcct tggcttcccg tgtgcggagg tgtccggcct gagcacggag 240

cgtgtccggg agctggctgt ggccttggca cagaagaatg tcaagctctc aacagagcag 300

ctgcgctgtc tggctcaccg gctctctgag ccccccgagg acctggacgc cctcccattg 360

gacctgctgc tattcctcaa cccagatgcg ttctcggggc cccaggcctg cacccatttc 420

ttctcccgca tcacgaaagc caatgtggac ctgctcccga gggggctccc cgagcgacag 480

cggctgctgc ctgcggctct ggcctgctgg ggtgtgcggg ggtctctgct gagcgaggct 540

gatgtgcggg ctctgggagg cctggcttgc gacctgcctg ggcgctttgt ggccgagtcg 600

gccgaagtgc tgctaccccg gctggtgagc tgcccgggac ccctggacca ggaccagcag 660

gaggcagcca gggcggctct gcagggcggg ggaccccctt acggcccccc gtcgacatgg 720

tctgtctcca cgatggacgc tctgcggggc ctgctgcccg tgctgggcca gcccatcatc 780

cgcagcatcc cgcagggcat cgtggccgcg tggcggcaac gctcctctcg ggacccatcc 840

tggcggcagc ctgaacggac catcctccgg ccgcggttcc ggcgg 885

<210> 7
<211> 30
<212> DNA
<213> Artificial

<220>
<223> primer No.5'-3

<400> 7
acgggatcca ggaccctggc tggagagaca 30

<210> 8
<211> 29
<212> DNA
<213> Artificial

<220>
<223> primer No.3'-3

<400> 8
aagctcgagc cgccggaacc gcggccgga 29

<210> 9
<211> 14
<212> PRT

<213>  Artificial

<220>
<223>  peptide(e)

<400>  9

Arg Gln Pro Glu Arg Thr Ile Leu Arg Pro Arg Phe Arg Arg
1               5                   10

## Description

### TECHNICAL FIELD

The present invention relates to a diagnostic agent, a diagnostic kit and a diagnostic method which can be used for the early diagnosis of mesothelioma.

### BACKGROUND ART

In recent years, the high incidence of mesothelioma in people who have exposed to asbestos has developed into social problem. Mesothelioma exists in both malignant and benign forms. Most patients with malignant mesothelioma have a history of exposure to asbestos, and typically develop malignant mesothelioma after a long period of latency of 30 to 35 years following exposure. Hence, the possibility of an increase in the number of mesothelioma patients in the future can not be denied.

Mesothelioma is a disease which is difficult to detect at early stage. It is currently being diagnosed by methods involving the use of magnetic resonance imaging (MRI) and computerized tomography (CT), however these methods have problems such as it is difficult to diagnose early stage by using these methods and when it is diagnosed as mesothelioma the illness has already advanced. Hence, there exists a strong desire for the early diagnosis of the disease, however effective early diagnostic markers have yet to be found.

Markers relating to mesothelioma which have been discovered to date are the proteins called Erc, MPF and mesothelin. The backgrounds of their discovery were as follows.
First, in 1994, Yamaguchi et al. reported that a protein called megakaryocyte potentiating factor (MPF) had been purified from the supernatant of the human pancreatic cancer cells HPC-Y5 (Non-Patent Document 1). In 1995, Kojima et al. reported that MPF had been cloned from the cDNA of the human pancreatic cancer cells HPC-Y5 (Non-Patent Document 2).

Later, Chang et al. made a monoclonal antibody K1 which reacts with a 40kDa epitope expressed at the surface of mesothelial cells, ovarian cancer and mesothelioma. They used the antibody to search for a gene that encodes the antigenic protein, and isolated a gene having a 1884bp open reading frame which codes for a 69kDa protein. In addition, Chang et al. showed that the 69kDa protein is the precursor of the above mentioned 40kDa protein, and that the 69kDa protein is cleaved by the action of phosphatidylinositol-specific phospholipase (PI-PL) to express a 40kDa protein at the cell surface. Chang et al. called the 69kDa protein mesothelin (Non-Patent Document 3). Subsequently, it became clear that the above-described MPF and mesothelin are the same protein.

In other research, Scholler et al. searched for a protein that is recognized by the monoclonal antibody OV569 made by immunizing ovarian cancer cells, discovered a protein with a molecular weight of 42 to 45kDa that has the same sequence as the membrane-binding region of mesothelin, and called it the "soluble member of mesothelin." It is appeared that this protein incurs a frameshift mutation during formation, as a result of which the GPI anchor portion in the C-terminal region acquires a different sequence, making the protein soluble. Scholler et al. constructed a Sandwich EIA assay system by using a different monoclonal antibody 4H3 which recognizes the same protein as OV569, and assayed the sera from patients with ovarian cancer. As a result, they discovered that the patients show a higher value for the protein than healthy subjects (Non-Patent Document 4). Robinson et al. have reported that measurements on patients with mesothelioma by using the assay system showed high values for the above mentioned protein compared to healthy subjects (Non-Patent Document 5).

The inventors (Hino et al.) have, in the course of years of research on animal models of disease, sought the causative genes in the Eker rat model of renal carcinogenesis. As a result, we discovered genes which are more highly expressed in cell lines derived from Eker rat renal carcinoma than in normal renal tissue, and called one of these genes Erc. As a result of searching for human homologs of the rat Erc gene, we showed that, in humans, MPF has 56.1% homology to rat Erc (Non-Patent Documents 6 and 7).

Based on the above findings, it is appeared that (1) human Erc, MPF and mesothelin (hereinafter collectively referred to as "Erc/MPF/mesothelin") are glycoproteins of 622 amino acids in all and have a RPRFRR sequence at amino acids 290 to 295 which may incur processing with a furin-like protease to be cleaved to 31kDa and 40kDa fragments, (2) the C-terminal region of the 40kDa fragment has a GPI anchor region at the C-terminus thereof and remains in a form bound to the cell membrane, whereas the 31kDa fragment on the N-terminal side is secreted as soluble protein (hereinafter referred to as "31 kDa secreted Erc/MPF/mesothelin"), and (3) the C-terminal region of the 40kDa fragment bonded to the cell membrane can also be released from the cell by phosphatidylinositol-specific phospholipase C (PI-PLC) treatment.

It is reported that the Erc/MPF/mesothelin identified by these different approaches is not only strongly expressed in normal mesothelial cells and mesotheliomas, but present in the blood of mesothelioma patients. However, it has not been shown from these findings which type of Erc/MPF/mesothelin is secreted in mesotheliomas.

The inventors have shown that the above-described 31 kDa secreted Erc/MPF/mesothelin is present in a high concentration within the sera of mesothelioma patients, and have reported that mesotheliomas can be diagnosed using this secreted protein (Non-Patent Document 8). However, this report does not visualize the state of the target protein in the patient sample. In this study, measurement was carried out using an antibody that recognizes the C-terminal region of the 31kDa secreted Erc/MPF/mesothelin, and thus only 31kDa secreted Erc/MPF/mesothelin with the entire length retained was measured; but degraded fragments which lost C terminal portion due to some activity could not be measured.

Following the reports by the inventors, it has been reported that mesotheliomas can be diagnosed by the detecting of 31 kDa secreted Erc/MPF/mesothelin (Non-Patent Document 9), but this report does not indicate the MPF values measured with an ELISA system as absolute values. Also, in this study, although the sera of mesothelioma patients and healthy subjects were measured, the results are indicated merely as absorbances instead of being presented as absolute values for MPF to make it impossible to compare values obtained from different tests. The reason for the above is presumably that conversion between standard substances and the MPF in blood cannot be achieved. And it appears likely that the underlying cause is that the antibodies have different specificities and affinities between to recombinant forms and to natural forms in the blood. This report investigated differences in the recognized epitopes between several antibodies obtained, however it merely tested whether the respective epitopes were different or not and did not identify the actual position of the recognized epitope for each antibody. Hence, this prior art was not appeared to be enough for use in practical diagnosis.

Non-Patent Document 1: Yamaguchi, N.; Hattori, K.; Oh-eda, M.; Kojima, T.; Imai, N.; Ochi, N.: "A novel cytokine exhibiting megakaryocyte potentiating activity from a human pancreatic tumor cell line HPC-Y5." J. Biol. Chem. 269:805-808 (1994). PMID: 8288629
Non-Patent Document 2: Kojima, T.; Oh-eda, M.; Hattori, K.; Taniguchi, Y.; Tamura, M.; Ochi, N.; Yamaguchi, N.: "Molecular cloning and expression of megakaryocyte potentiating factor cDNA." J. Biol. Chem. 270:21984-21990 (1995). PubMed ID: 7665620
Non-Patent Document 3: Chang, K.; Pastan, I.: "Molecular cloning of mesothelin, a differentiation antigen present on mesothelium, mesotheliomas, and ovarian cancers." Proc. Nat. Acad. Sci. 93:136-140 (1996). PubMed ID: 8552591
Non-Patent Document 4: Scholler, N.; Fu, N.; Yang, Y.; Ye, Z.; Goodman, G.E.; Hellstrom, K.E.; Hellstrom, I.: "Soluble member(s) of the mesothelin/megakaryocyte potentiating factor family are detectable in sera from patients with ovarian carcinoma." Proc. Nat. Acad. Sci. 96:11531-11536 (1999). PubMed ID: 10500211
Non-Patent Document 5: Robinson, B.W.; Creaney, J.; Lake, R.; Nowak, A.; Musk, A.W.; de Klerk, N.; Winzell, P.; Hellstrom, K.E.; Hellstrom, I.: "Soluble mesothelin-related protein-A blood test for mesothelioma." Lung Cancer 49 Suppl.1:S109-111 (2005). PMID: 15950789
Non-Patent Document 6: Hino, O.; Kobayashi, E.; Nishizawa, M.; Kubo, Y.; Kobayashi, T.; Hirayama, Y.; Takai, S.; Kikuchi, Y.; Tsuchiya, H.; Orimoto, K. et al.: "Renal carcinogenesis in the Eker rat." J. Cancer Res. Clin. Oncol. 121:602-605 (1995). PMID: 7559744.
Non-Patent Document 7: Yamashita, Y.; Yokoyama, M.; Kobayashi, E.; Takai, S.; Hino, O.: "Mapping and determination of the cDNA .sequence of the Erc gene preferentially expressed in renal cell carcinoma in the Tsc2 gene mutant (Eker) rat model." Biochem. Biophys. Res. Commun. 275(1):134-140 (2000). PMID: 10944454
Non-Patent Document 8: Shiomi, K.; Miyamoto, H.; Segawa, T.; Hagiwara, Y.; Ota, A.; Maeda, M.; Takahashi, K.; Masuda, K.; Sakao, Y.; Hino, O.: "Novel ELISA system for detection of N-ERC/mesothelin in the sera of mesothelioma patients." Cancer Sci. 97:928-932 (2006). PMID: 16776777
Non-Patent Document 9: Onda, M.; Nagata, S.; Ho, M.; Bera, T.K.; Hassan, R.; Alexander, R.H.; Pastan, I.: "Megakaryocyte potentiation factor cleaved from mesothelin precursor is a useful tumor marker in the serum of patients with mesothelioma." Clin. Cancer Res. 12:4225-4231 (2006). PMID: 16857795

### DISCLOSURE OF THE INVENTION

The present invention provides a diagnostic agent and a diagnostic kit for mesothelioma, which are able to effectively distinguish mesothelioma patients from healthy subjects, to screen mesothelioma patients from patients who have exposed to asbestos, and to distinguish mesothelioma patients from patients with other diseases such as lung cancer at a high sensitivity compared to previously reported mesothelioma diagnostic kits.

As a result of meticulous searches using bodily fluids from mesothelioma patients, the inventors have discovered that the bodily fluids contain both 31kDa secreted Erc/MPF/mesothelin and decomposed fragments thereof. The inventors also found that fragments of 31kDa secreted Erc/MPF/mesothelin were not detected by assay systems which employ antibodies obtained by using polypeptides as antigens which include the N-terminal and C-terminal amino acids of 31kDa secreted Erc/MPF/mesothelin, which is antigen sites commonly used in antibody production in the past.

In addition, the inventors have achieved extensive investigations on assay systems capable of measuring both the 31kDa secreted Erc/MPF/mesothelin and fragments thereof, and discovered that by selecting antibodies which recognize appropriate sites on 31kDa secreted Erc/MPF/mesothelin, both of the 31kDa secreted Erc/MPF/mesothelin and fragments thereof can be measured at a high sensitivity. The inventors accomplished the present invention by enabling more precise diagnosis of mesothelioma than in the past.

The present invention provides an antibody which recognizes a peptide with a deletion of an amino acid from the C-terminus of 31kDa secreted Erc/MPF/mesothelin, and specifically provides an antibody which recognizes an N-terminal side fragment from among the fragments formed by leaving 31kDa secreted Erc/MPF/mesothelin in a bodily fluid at room temperature. The invention also provides a diagnostic agent for mesothelioma which includes as the active ingredient an antibody that recognizes a peptide with a deletion of an amino acid from the C-terminus of 31kDa secreted Erc/MPF/mesothelin, and specifically provides a diagnostic agent for mesothelioma which includes as the active ingredient an antibody that recognizes an N-terminal side fragment from the fragments formed by leaving 31kDa secreted Erc/MPF/mesothelin in a bodily fluid at room temperature.

The invention further provides a diagnostic kit for mesothelioma which includes an antibody that recognizes a peptide with a deletion of amino acid(s) from the C-terminus of 31kDa secreted Erc/MPF/mesothelin, and specifically a diagnostic kit for mesothelioma which includes a first reagent containing a first antibody that recognizes a peptide with a deletion of amino acid(s) from the C-terminus of 31kDa secreted Erc/MPF/mesothelin in combination with a second reagent containing a second antibody that recognizes a peptide with a deletion of amino acid(s) from the C-terminus of 31kDa secreted Erc/MPF/mesothelin and has a recognition site different from that of the first antibody.

The invention still further provides a method of diagnosing mesothelioma which is **characterizes in that** the step of determining the amount of 31kDa secreted Erc/MPF/mesothelin in a sample with the above mentioned diagnostic kit for mesothelioma, and the step of using the determined amount as an indicator.

The antibody of the invention is able to detect an N-terminal side fragment from the fragments formed from 31kDa secreted Erc/MPF/mesothelin by various causes as well as 31kDa secreted Erc/MPF/mesothelin.

By using the assay system of the present invention, it is possible to measure the amount of 31kDa secreted Erc/MPF/mesothelin which was originally present in the body, and to diagnose a mesothelioma and determine the degree of progress with a high degree of accuracy compared with assay systems that have been previously used. Moreover, by using the said assay system, measurement can be carried out at a high sensitivity regardless of the conditions of sample collection and storage.

### BRIEF DESCRIPTION OF THE DRAWINGS

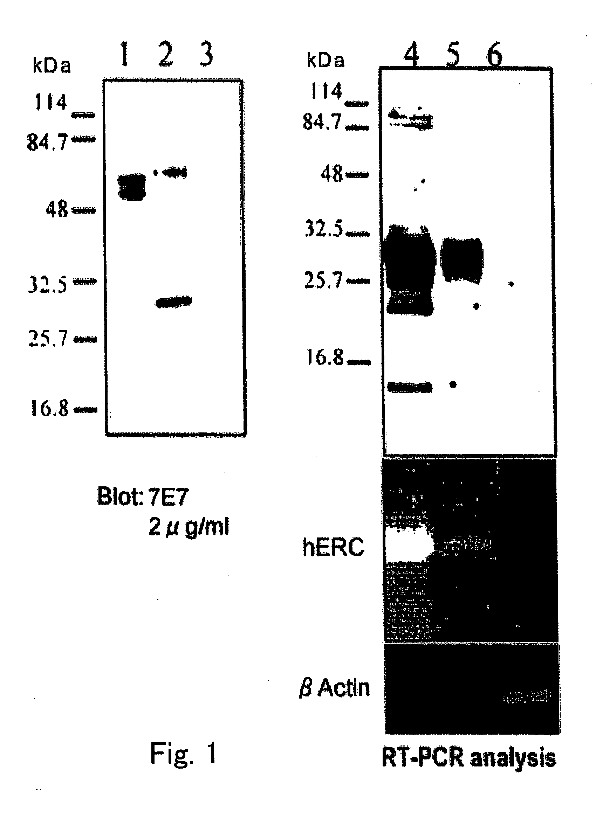

FIG. 1 shows the results of specificity tests by Western blotting on monoclonal antibody 7E7 (Lane 1 shows the results obtained for a sample of a lysate of COS-1 cells transduced with cDNA for the full-length human Erc/MPF/mesothelin, Lane 2 shows the results obtained for a sample of a culture supernatant of COS-1 cells transduced with cDNA for the full-length human Erc/MPF/mesothelin, Lane 3 shows the results obtained for a sample of a culture supernatant of COS-1 cells transduced only with the vector, and Lanes 4, 5 and 6 show the results obtained for a sample of culture supernatants of HeLa cells, MKN-28 cells and NRC-12 cells, respectively).
FIG. 2 shows the results of specificity tests by Western blotting on monoclonal antibody 16K16 (Lane 1 shows the results obtained for a sample of a culture supernatant of CHO cells transduced with cDNA for the full-length human Erc/MPF/mesothelin, Lane 2 shows the results obtained for a sample of a culture supernatant of CHO cells not transduced with a gene, and Lanes 3 and 4 show the results obtained for a sample of lysates of the respective cells).
FIG. 3 shows the results of recognition site identification tests of monoclonal antibody 7E7 by Western blotting (the cross marks in the figure indicate false negatives).
FIG. 4 shows the results of recognition site identification tests of monoclonal antibody 16K16 by Western blotting (the cross marks in the figure indicate false negatives).
FIG. 5 shows the results of specificity tests of polyclonal antibody 4 by Western blotting (the sample in each lane is the same as in FIG. 1).
FIG. 6 shows the results of assays of the concentration of 31kDa secreted Erc/MPF/mesothelin in the pleural effusion of a mesothelioma patient using various antibodies (in the figure, I to VIII indicate the antibody combinations used in the ELISA kits (I indicates a combination of 4 and 34, II indicates a combination of 211 and 34, III indicates a combination of 7E7 and 34, IV indicates a combination of 211 and 4, V indicates a combination of 16K16 and 4, VI indicates a combination of 7E7 and 4, VII indicates a combination of 34 and 4, and VIII indicates a combination of 7E7 and 16K16)).
FIG. 7 shows a typical standard curve created by using the 7E7 - 16K16 kit.
FIG. 8 shows the concentration of 31kDa secreted Erc/MPF/mesothelin in the culture supernatant of cultured cells, as measured using the 7E7 - 16K16 kit.
FIG. 9 shows a typical standard curve created using the 7E7 - 4 kit.
FIG. 10 shows the concentration of 31kDa secreted Erc/MPF/mesothelin in the culture supernatant of cultured cells, as measured using the 7E7 - 4 kit.
FIG. 11 shows the concentration of 31kDa secreted Erc/MPF/mesothelin in the sera of healthy subjects, as measured using the 7E7 - 4 kit.

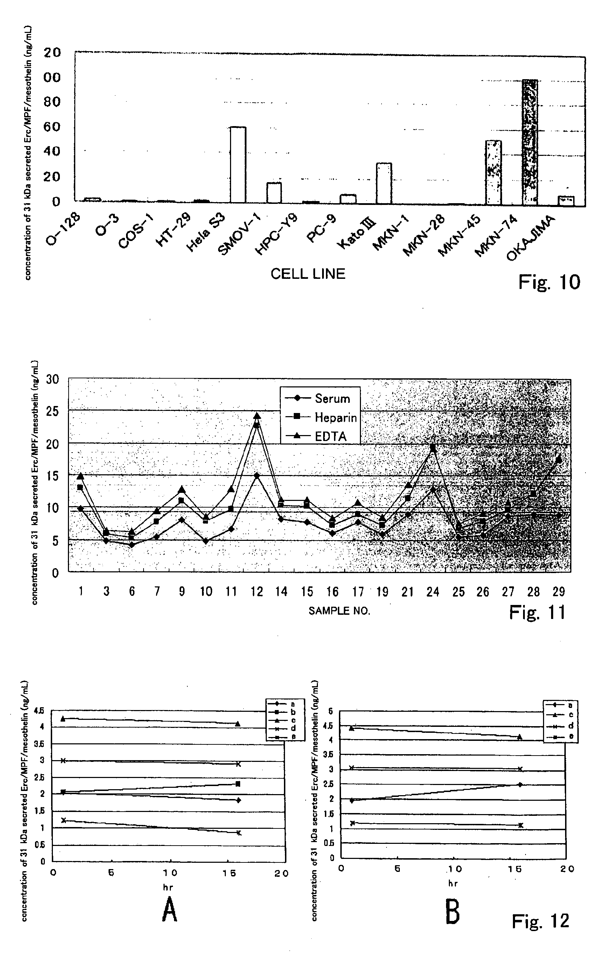

FIG. 12 shows the concentration of 31kDa secreted Erc/MPF/mesothelin in sera, as measured using the 7E7 - 16K16 kit (in the figures, A shows the results measured for samples prepared by collecting blood sample, leaving the blood sample at room temperature for one hour, then leaving the blood sample at room temperature for additional 16 hours, subsequently centrifuging the blood sample to separate off the serum, and then freezing the serum; B shows the results measured for samples prepared by collecting blood sample, leaving the blood sample at room temperature for one hour, then centrifuging the blood sample to separate off the serum, subsequently leaving the serum at room temperature for 16 hours, and then freezing the serum).

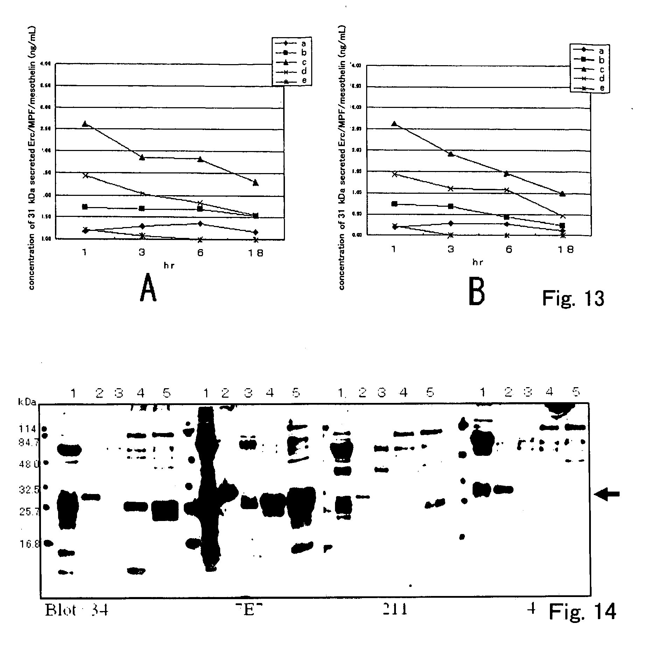

FIG. 13 shows the concentration of 31kDa secreted Erc/MPF/mesothelin in serum, as measured using the 7E7 - 4 kit (in the figures, A shows the results measured for samples prepared by collecting blood sample, leaving the blood sample at room temperature for one hour, then leaving the blood sample at room temperature for additional 16 hours, subsequently centrifuging the blood sample to separate off the serum, and freezing the serum; B shows the results measured for samples prepared by collecting blood sample, leaving the blood sample at room temperature for one hour, then centrifuging the blood sample to separate off the serum, subsequently leaving the serum at room temperature for 16 hours, then freezing the serum).
FIG. 14 shows the results of Western blotting with monoclonal antibody 7E7 and polyclonal antibodies 34, 211 and 4 using the pleural effusion from a mesothelioma patient (in the figure, Lane 1 shows the results obtained for a sample of untreated pleural effusion from mesothelioma patient, Lane 2 shows the results for a sample of the fraction that adsorbed to a C-terminal antibody column, Lane 3 shows the results for a sample of the fraction that did not adsorb to an N-terminal, C-terminal antibody column, Lane 4 shows the results for a sample of the fraction that did not adsorb to an Antibody 7E7 column, and Lane 5 shows the results for a sample of the fraction that adsorbed to an Antibody 7E7 column; the arrow indicates 31kDa).

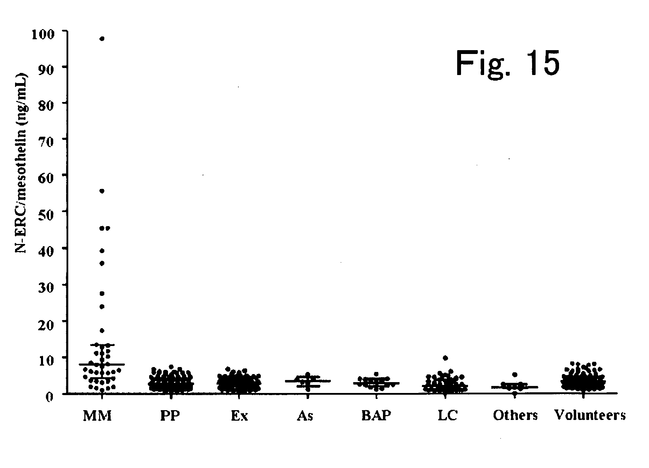

FIG. 15 is a diagram showing the results of measurement, using an ELISA system (7E7 - 16K16 kit), of the concentration of 31kDa secreted Erc/MPF/mesothelin in the sera of mesothelioma patients, healthy subjects, patients with asbestos-related diseases/asbestos exposed subjects (pleural plaque patients, exposed subjects, asbestosis patients, benign asbestos pleurisy patients), lung cancer patients, and patients with other diseases to be distinguished from mesothelioma. In the figure, on the horizontal axis, "MM" means mesothelioma patients, "PP" means plural plaque patients, "Ex" means patients with a history of exposure to asbestos, "As" means asbestosis patients, "BAP" means benign asbestos pleurisy patients, "LC" means lung cancer patients, "Others" means patients with other diseases to be distinguished from mesothelioma, and "Volunteers" means healthy subjects. The vertical axis represents the amount (ng/mL) of Erc/MPF/mesothelin detected.

### BEST MODE FOR CARRYING OUT THE INVENTION

In this specification, the "peptide obtained by deletion of amino acid(s) from the C-terminus of 31kDa secreted Erc/MPF/mesothelin" (abbreviated below as the "N-fragment") is not limited as long as it is a peptide obtained by deletion of an amino acid from the C-terminus of the 31kDa secreted Erc/MPF/mesothelin having the amino acid sequence of SEQ ID NO: 1. In SEQ ID NO: 1, the sequence from the first amino acid methionine to the 33^{rd} amino acid proline is a signal sequence; a peptide which excludes the amino acids of the signal sequence is preferable as the N-fragment. The number of C-terminal amino acids deleted in the N-fragment is not limited but at least one, and may be, for example, 5 or more, 10 or more, 15 or more, 20 or more, 25 or more, 30 or more, 35 or more, or 40 or more. The number of amino acids deleted from the C-terminus in the N-fragment does not have any particular upper limit, and may be, for example, 155, 150, 100, 90, 80, 70, 65 or 60. For example, N-fragment may be N-terminal side fragments of the fragments formed by leaving 31kDa secreted Erc/MPF/mesothelin in a bodily fluid at room temperature. The bodily fluid used includes, but is not limited to, for example, blood, serum, plasma, pleural effusion, urine and ascites. The bodily fluid is preferably blood and blood serum. The length of time to be incubated is not limited but preferably at least 1 hour, more preferably at least 2 hours, and most preferably at least 3 hours. In this specification, for example, the N-fragment includes more than one N-terminal side fragments in different lengths of the fragments formed by leaving 31kDa secreted Erc/MPF/mesothelin having the amino acid sequence in SEQ ID NO: 1 (this sequence corresponds to amino acids 1 to 295 of the amino acid sequence of human Erc/MPF/mesothelin in Accession No. AAV87530) in blood or blood serum at room temperature for at least 3 hours. The N-fragment in the present specification is preferably not recognized by antibodies that are conventionally produced using polypeptides with C-terminal amino acid as the antigens which is used in ordinary antibody production. In this specification, the N-fragment is more preferably a fragment that arises from the degradation of 31kDa secreted Erc/MPF/mesothelin, specifically a fragment having the sequence of amino acids 34 to 229 (preferably amino acids 34 to 139, and more preferably amino acids 68 to 139) of the amino acid sequence of 31kDa secreted Erc/MPF/mesothelin (SEQ ID NO: 1).

In the present specification, "mesothelioma" refers to a malignant tumor that originates in mesothelial cells. Specifically, for example, mesothelioma includes epithelial mesothelioma, sarcomatous mesothelioma and biphasic mesothelioma.

The antibody of the present invention is not limited as long as it is an antibody which recognizes the N-fragment. A plurality of fragments may be present as N-fragments, and it is not necessary for the antibody of the present invention to recognize all of the fragments and the antibody may recognize only some portion of the fragments so long as it can be used as a diagnostic agent for mesothelioma. The antibody of the present invention is preferably an antibody which recognizes two or more N-fragments. The antibody of the invention includes, for example, antibodies which recognize an N-terminal side fragment of fragments formed by leaving 31kDa secreted Erc/MPF/mesothelin in blood or blood serum at room temperature for at least 3 hours. The antibody of the present invention is more preferably an antibody which recognizes both 31kDa secreted Erc/MPF/mesothelin and an N-fragment. Such antibodies include, for example, antibodies which recognize the amino acid sequences preserved in 31kDa secreted Erc/MPF/mesothelin and N-fragments. Such recognition sites include, for example, following amino acid sequences (a) to (d) (SEQ ID NOS: 2 to 5), are preferably (a) to (c), and more preferably (b) and (c).
(a) SRTLAGETGQEAAPLDGV (SEQ ID NO: 2, which is the sequence of amino acids 34 to 51 in SEQ ID NO: 1)
(b) GFPCAE (SEQ ID NO: 3, which is the sequence of amino acids 68 to 73 in SEQ ID NO: 1)
(c) PQACTH (SEQ ID NO: 4, which is the sequence of amino acids 134 to 139 in SEQ ID NO: 1)
(d) CPGPLDQDQQEAARAALQG (SEQ ID NO: 5, which is the sequence of amino acids 211 to 229 in SEQ ID NO: 1)

The source of the antibody of the present invention is not limited, and not necessarily human origin. The antibody of the present invention may be either a polyclonal antibody or a monoclonal antibody, and a monoclonal antibody is preferred due to the ability to identify the recognition site.

The antibody of the present invention may be prepared by preparing antibodies in accordance with a method known to those skilled in the art, for example, the methods described in *Zoku Seikagaku Jikken Ko̅za* [More lectures on experimental biochemistry]: "Meneki-Seikagaku Kenkyu̅ Ho̅ [Research methods in immunobiochemistry]" (Japanese Biochemical Society) using a part of or all of 31kDa secreted Erc/MPF/mesothelin as the antigen, and then select the antibody by screening with a polypeptide which is identical with some portion of the amino acid sequence preserved on the N-fragment.

The part of or all of 31kDa secreted Erc/MPF/mesothelin that is used as the antigen to produce the antibody of the present invention includes polypeptides which are identical with some part of or all of amino acids 1 to 295, preferably amino acids 35 to 295, of the amino acid sequence of SEQ ID NO: 1 synthesized by a synthesizing machine, or produced by inserting a part of or all of the cDNA (SEQ ID NO: 6) coding for said amino acid sequence into a vector, transforming a host microorganism (e.g., *Escherichia coli*) or cultured cells with said vector, culturing the transformed host microorganism or cultured cells (e.g., *E. coli*) to produce a recombinant protein or polypeptide, and then purifying said recombinant protein or polypeptide with an affinity column or nickel column. A polynucleotide which is identical with a part of or all of the cDNA coding for the said amino acid sequence may be obtained by cloning from a cDNA library of, for example, human tumor cell lines using mammalian Erc/MPF/mesothelin cDNA such as rat Erc/MPF/mesothelin cDNA as the probe.

More specifically, the procedure for preparing the antibody of the present invention is as follows. To prepare the antibody of the present invention as a polyclonal antibody, first, a polynucleotide coding for the cDNA sequence (SEQ ID NO: 6) of human 31kDa secreted Erc/MPF/mesothelin is prepared by the polymerase chain reaction (PCR). Next, the polynucleotide is inserted into a vector such as pGEX, and the vector is introduced into a host microorganism such as *E. coli,* which is then grown on a LB medium or the like, thereby producing recombinant proteins of the polypeptide. The resulting recombinant proteins are then dissolved as the antigen in a sodium phosphate buffer solution (PBS), followed by conjugation with an adjuvant such as Freund's complete adjuvant or incomplete adjuvant or alum, and the resulting conjugate is used as an immunogenic agent to immunize mammals or other animals.

The animals which are immunized may be ones that are commonly used in the field of which the present invention, such as mice, rats, rabbits, goats, horses or chickens. The method used to administer the immunogenic agent at the time of immunization may be hypodermic injection, intraperitoneal injection, intravenous injection, hypodermic injection or intramuscular injection and preferably hypodermic injection or intraperitoneal injection. Immunization may be carried once or several times at suitable intervals, preferably of 1 to 5 weeks.

Finally, blood is collected from the immunized animal in accordance with a conventional method, and then the serum is separated from the collected blood and subjected to antigen-specific purification by affinity column chromatography using a column on which the recombinant protein used as the antigen is immobilized, thereby enabling to obtain an antibody of the present invention as polyclonal antibody.

Alternatively, preparation of the antibody of the present invention as a monoclonal antibody may be carried out in accordance with a conventional method of preparing monoclonal antibodies, such as the methods described in Kopepuchido Kotai Jikken Purotokoru [Experimental protocols for anti-peptide antibodies] by S. Omi, K. Tsujimura and M. Inagaki (Shujunsha, 1994) or in Tankuron Kotai Jikken Manyuaru [Experimental manual on monoclonal antibodies] edited by S. Toyama and M. Ando (Kodansha, 1987), by fusing immune cells obtained from immunized animal by the above mentioned recombinant protein with myeloma cells to obtain a hybridoma, and then collecting antibody from a culture of said hybridoma. The antibody collected is then screened by an EIA method using a 96-well microtiter plate with immobilized recombinant protein or synthesized polypeptide having the above-indicated amino acid sequence which was used as the antigen, thereby enabling to obtain an antibody of the present invention as monoclonal antibody.

More specifically, the more preferred monoclonal antibody of the present invention included in the diagnostic kit of the present invention as an active ingredient recognizes the following amino acid sequence (b) or (c). Said monoclonal antibody may be obtained by screening the antibodies collected from a culture of the above-described hybridoma by an EIA method using a microtiter plate with immobilized antigen of following amino acid sequence (a) or (b).
(b) GFPCAE (SEQ ID NO: 3, which is the sequence of amino acids 68 to 73 in SEQ ID NO: 1)
(c) PQACTH (SEQ ID NO: 4, which is the sequence of amino acids 134 to 139 in SEQ ID NO: 1)

The antibody of the present invention obtained may be labeled or immobilized, if needed to be used as the diagnostic agent of the present invention. Labeling is carried out by fusing with an enzyme such as horseradish peroxidase (abbreviated below as "HRP") or alkaline phosphatase, a fluorescent substance such as fluorescein isocyanate or Rhodamine, a radioactive substance such as ³²P or ¹²⁵I, or a labeling substance such as a chemiluminescent substance. Immobilization is carried out by binding the antibody of the present invention to a suitable solid phase. The solid phase used may be any solid phase commonly employed in immunochemical assay methods, and can be, for example, plates such as 96-well polystyrene microtiter plates and aminated microtiter plates, and various types of beads. Immobilization of the antibody of the present invention may be achieved by, for example, adding a buffer solution containing the antibody to the carrier, and leaving them.

The diagnostic kit for mesothelioma of the present invention (hereinafter referred to as "present invention kit") may be manufactured in accordance with a conventional method by using the antibody of the present invention which may be labeled or immobilized if needed with a combination of other materials such as a buffer solution for dilution, a standard substance, a buffer solution for the substrate, a reaction stopping solution and a washing solution. The diagnostic kit for mesothelioma of the present invention preferably includes two types of antibodies that recognize peptides which lack C-terminal amino acids from 31kDa secreted Erc/MPF/mesothelin, and more preferably includes two types of antibodies of different recognition sites that recognize peptides which lack C-terminal amino acids from 31kDa secreted Erc/MPF/mesothelin. Specifically, two types of the antibody of the present invention are used in manufacturing the present invention kit, as a combination of a first reagent (e.g., an immobilized reagent) containing a first antibody that recognizes a peptide which lack C-terminal amino acid(s) from 31kDa secreted Erc/MPF/mesothelin, and a second reagent (e.g., a labeled antibody) comprising a second antibody that recognizes a peptide which lack C-terminal amino acid from 31kDa secreted Erc/MPF/mesothelin, wherein the second antibody recognizes a different recognition site from the first antibody.

The combination of the first antibody and the second antibody used in the present invention kit, is not limited but includes a combination of antibodies whose recognition sites are included in, the sequence of amino acids 34 to 229, preferably the sequence of amino acids 34 to 139, and more preferably the sequence of amino acids 68 to 139 of the total amino acid sequence (SEQ ID NO: 1) of 31kDa secreted Erc/MPF/mesothelin. Combinations of the first antibody and the second antibody include combinations of antibodies, each of which recognizes any one of the amino acid sequences in following (a) to (d) (SEQ ID NOS: 2 to 5), and are preferably the combination of an antibody which recognizes the amino acid sequence in (a) or (b) with an antibody which recognizes the amino acid sequence in (c) or (d), more preferably the combination of an antibody which recognizes the amino acid sequence in (a) or (b) with an antibody which recognizes the amino acid sequence in (c) and still more preferably the combination of (b) and (c).
(a) SRTLAGETGQEAAPLDGV (SEQ ID NO: 2, which is the sequence of amino acids 34 to 51 in SEQ ID NO: 1)
(b) GFPCAE (SEQ ID NO: 3, which is the sequence of amino acids 68 to 73 in SEQ ID NO: 1)
(c) PQACTH (SEQ ID NO: 4, which is the sequence of amino acids 134 to 139 in SEQ ID NO: 1)
(d) CPGPLDQDQQEAARAALQG (SEQ ID NO: 5, which is the sequence of amino acids 211 to 229 in SEQ ID NO: 1)

The contained antibody of the present invention enables the diagnostic agent and kit of the present invention produced as described above to assay not only 31kDa secreted Erc/MPF/mesothelin, but also fragments thereof formed due to a variety of causes in various bodily fluids of mesothelioma patients.

Illustrative examples of methods for detecting the presence and measuring the levels of both 31kDa secreted Erc/MPF/mesothelin and fragments thereof using the diagnostic agent and diagnostic kit of the present invention include various methods used in ordinary immunochemical assays, such as radioisotope immunoassays (RIA method), the ELISA method (E. Engvall. et al.: Methods in Enzymol. 70, 419-439 (1980)), the fluorescent antibody method, the plaque method, the spot method, the agglutination method, the Ouchterlony and immunochromatography (Haiburidoma Ho to Monokuronaru Kotai [Hybridoma methods and monoclonal antibodies], (published by R&D Planning; March 5, 1982), pp. 30-53).

Although any of these assay methods may be selected as appropriate according to various considerations, the use of the ELISA method is preferred from the standpoint of sensitivity and convenience. By way of illustration, the procedure involved in assaying 31kDa secreted Erc/MPF/mesothelin and fragment thereof using the sandwich method, which is one of an ELISA method, is described below.

First, in Step (A), a first.antibody which recognizes a peptide which lacks C-terminal amino acid(s) from 31kDa secreted Erc/MPF/mesothelin (hereinafter referred to as the "first antibody") is immobilized on a carrier. Next, in Step (B), the carrier surface on which antibody is not immobilized is blocked with a protein or other substance unrelated to the first antibody. In Step (C), samples containing 31kDa secreted Erc/MPF/mesothelin and/or a N-fragment thereof in various concentrations are added to the carrier so as to form complexes of the 31kDa secreted Erc/MPF/mesothelin and/or the N-fragment with the first antibody. Then in step (D), a second antibody which recognizes a peptide which lacks C-terminal amino acid(s) from 31kDa secreted Erc/MPF/mesothelin at a different site from the immobilized first antibody (hereinafter referred to as the "second antibody") is added so as to bond with the complex. Finally, in Step (E), the labeled amount of complex is measured, and the total amount of 31kDa secreted Erc/MPF/mesothelin and N-fragments in the sample can be given by using a predetermined standard curve.

Specifically, in Step (A), the carrier used for immobilizing the first antibody is not limited but may be any carrier commonly used in immunochemical assay methods. Such carriers include, for example, 96-well polystyrene microtiter plates and aminated microtiter plates. The first antibody may be immobilized by, for example, adding a buffer solution containing the antibody to the carrier and leaving them. A commonly known buffer solution may be used for this purpose, such as 10mM PBS. The concentration of the antibody in the buffer solution may be selected from a wide range, but is typically in the range of from 0.01 to 100 microgram/mL, and preferably in the range of from 0.1 to 20 microgram/mL. When a 96-well microtiter plate is used as the carrier, it is desirable that the capacity per well is less than 300 microliter/well, and preferably in the range of from 20 to 150 microliter/well. The incubation conditions are not limited but overnight incubation at about 4°C is generally appropriate.

The blocking of step (B) is employed in order to prevent the carrier to which the first antibody is immobilized in Step (A) from remaining areas where subsequently added 31kDa secreted Erc/MPF/mesothelin and/or N-fragments thereof can adhere independent of the antibody-antigen reaction. The blocking agent used may be, for example, BSA or a skim milk solution, or a commercial blocking agent such as Block-Ace (Danippon Pharmaceutical Co., Ltd.; Code No. UK-25B). The procedure of blocking includes, but not limited to, for example, adding a suitable amount of Block-Ace to the area where the antigen is immobilized, leaving overnight at about 4°C, and then rinsing with a buffer solution.

In the subsequent Step (C), a sample containing 31kDa secreted Erc/MPF/mesothelin and/or N-fragments thereof is contacted with the immobilized first antibody, and the 31kDa secreted Erc/MPF/mesothelin and/or N-fragments thereof contained in the sample is captured by the immobilized first antibody, thereby forming a complex. The conditions for enabling the formation of such a complex is not limited but carried out by reaction for about 1 hour to overnight at about 4°C to about 37°C. After the reaction completion, it is preferable to rinse the carrier with a buffer solution so as to remove unreacted protein and the like. The buffer solution used in this reaction is preferably composed of 10mM PBS (pH 7.2) and 0.05% (v/v) Tween 20.

Next, in Step (D), the labeled second antibody recognizing the site that differs from the recognition site of the immobilized first antibody is added to the complex of the immobilized first antibody and the 31kDa secreted Erc/MPF/mesothelin and/or N-fragment thereof, thereby forming a complex of an immobilized first antibody - 31kDa secreted Erc/MPF/mesothelin and/or N-fragments thereof - labeled second antibody. After this reaction, it is preferable to rinse the carrier with buffer solution so as to remove unreacted protein and the like. The buffer solution used in the reaction may be the same as that described above. The amount of the labeled second antibody used in the Step (D) is about 5,000 to 10,000 times as much as the immobilized first antibody, and preferably an amount diluted in order that the final absorbance is 1.5 to 2.0. A buffer solution may be used for dilution. The reaction conditions is not limited but preferably carried out at about 4°C to 37°C for about 1 hour followed by rinsing with a buffer solution. By the above reaction, an immobilized first antibody - 31kDa secreted Erc/MPF/mesothelin and/or N-fragments thereof - labeled second antibody complex can be formed.

Finally, in Step (E), a chromogenic substrate solution which can react with the labeled substance is added to the immobilized first antibody - 31kDa secreted Erc/MPF/mesothelin and/or N-fragments thereof - labeled second antibody complex, and the absorbance is measured. In using peroxidase as the labeling substance in the above reaction, a chromogenic substance solution containing, for example, hydrogen peroxide and 3,3',5,5'-tetramethylbenzene (hereinafter, abbreviated as "TMB") may be used. The absorbance may typically be measured by adding the chromogenic substrate solution to the complex of the immobilized first antibody - 31kDa secreted Erc/MPF/mesothelin and/or N-fragments thereof - labeled second antibody, reacting at about 25°C for about 30 minutes, then adding 1 to 2N sulfuric acid to stop the enzyme reaction, and measuring the absorbance at a wavelength of 450nm. Alternatively, in the case of using alkaline phosphatase as the labeling substance, a preferred method employs developing color using p-nitrophenyl phosphate as the substrate, adding 2N sodium hydroxide to stop the enzyme reaction, and measuring the absorbance at 415 nm.

The amount of 31kDa secreted Erc/MPF/mesothelin and N-fragments in the sample can be calculated by employing a predetermined standard curve prepared using 31kDa secreted Erc/MPF/mesothelin of a known concentration by the above-described sandwich method and the like.

By using the above-mentioned assay methods, it is possible to calculate the amount of 31kDa secreted Erc/MPF/mesothelin and N-fragments in a sample or the amount of 31kDa secreted Erc/MPF/mesothelin originally present in the body. The calculation of the amount enables to diagnose a mesothelioma patient from a healthy subject, to distinguish between the success and failure of a surgical operation on a mesothelioma patient, and to predict the recurrence of a mesothelioma. A sample used in the diagnosis of mesothelioma are not limited as long as it contains 31kDa secreted Erc/MPF/mesothelin and/or N-fragment, and includes bodily fluids such as whole blood, serum, plasma, urine, lymphatic fluid, ascites and pleural effusion. In these samples, serum, plasma, ascites or pleural effusion is preferred.

Specifically, diagnosis of mesothelioma is performed by using the amount of 31kDa secreted Erc/MPF/mesothelin and N-fragments present in the sample as an indicator.
If this amount of a subject is judged to be significantly higher than the average value for healthy subjects, the subject can be diagnosed with mesothelioma. Indicators for judging that the amount of 31kDa secreted Erc/MPF/mesothelin and N-fragment in a sample is significantly higher than the average value in healthy subjects are not limited but includes employment of the statistical cutoff value, wherein the cutoff value is the mean value plus twofold standard deviation of the amount of 31kDa secreted Erc/MPF/mesothelin and N-fragment observed in the population of sera from healthy subjects, and preferably the mean value plus fivefold standard deviation of the amount of 31kDa secreted Erc/MPF/mesothelin and N-fragment observed in the population of sera from healthy subjects. Also, the above diagnosis of mesothelioma may employ the concentration of 31kDa secreted Erc/MPF/mesothelin and N-fragment in the sample as the indicator instead of the amount of 31kDa secreted Erc/MPF/mesothelin and N-fragment in the sample.

The result of surgical procedure on the mesothelioma may be evaluated and the recurrence of mesothelioma may be predicted based on the idea that the amount of 31kDa secreted Erc/MPF/mesothelin and N-fragment in samples from the mesothelioma patient changes depending on the presence of mesothelioma cells. Specifically, the removal of mesothelioma cells by the surgical operation can be confirmed by measuring the amount of 31kDa secreted Erc/MPF/mesothelin and N-fragment in samples obtained before and after the surgical operation and comparing the amount in sample obtained before the operation and that after the operation. A significant decrease of the amount in sample obtained after the operation from the amount in sample obtained before the operation means that mesothelioma cells have been removed by the surgical operation. Also, by monitoring the level of 31 kDa secreted Erc/MPF/mesothelin and N-fragment in samples following a surgical operation on mesothelioma, it is possible to determine that mesothelioma cells are present; i.e., to predict a relapse in the case that the level become higher.

By measuring the amount of 31kDa secreted Erc/MPF/mesothelin and N-fragment in samples using the diagnostic agent and kit of the present invention, it is possible, as described above, to diagnose mesothelioma patients from healthy subjects, to determine whether a surgical operation on a mesothelioma patient was successful or not, and to predict the recurrence of mesothelioma as well as to screen mesothelioma patients from subjects exposed to asbestos, and to distinguish such patients from patients with other diseases such as lung cancer. The incidence of pleural thickening, benign pleurisy, pulmonary fibrosis and lung cancer in persons who were exposed to asbestos is higher than that in healthy subjects. However, in patients with such diseases, the measured levels of 31kDa secreted Erc/MPF/mesothelin are significantly lower than that in mesothelioma patients. This enables to distinguish the subjects exposed to asbestos from mesothelioma patients. This is very useful for the optimization of treatment methods. A distinction of mesothelioma patients from, for example, lung cancer patients enables entirely different therapeutic approach to the each patient. Clinicians can avoid the useless administration of ineffective anticancer drugs which inflict pain on patients. The present invention enables an approach for tailored medication that has drawn attention recently.

The invention is illustrated in more detail below as examples, although these examples do not in any way limit the scope of the invention.

### Example 1: Preparation of Monoclonal Antibody (1)

### (1) Preparation of Antigen for immunization

The nucleotides corresponding to the amino acids consisting of the 35 to 295 in the amino acid sequence of 31 kDa secreted Erc/MPF/mesothelin shown as SEQ ID NO: 1 (i.e., nucleotides consisting of the region from 103 to 885 in the base sequence of SEQ ID NO: 6) were amplified by a polymerase chain reaction (PCR) using Primers No. 5'-3 and No. 3'-3 shown below as primers and fully human Erc/MPF/mesothelin cDNA (Accession No. AY743922) as a template. Next, a sequence coding for a GST (glutathione S-transferase) tag were added to the oligonucleotide obtained as described above and a sequence coding for a histidine tag were added to the C-terminal region of said oligonucleotide. The GST - 31kDa secreted mesothelin - His region obtained was inserted in pGEX-6P-1 (Amersham Biosciences) to give an expression vector, which was then integrated into E. *coli* as the expression vector to express a fusion protein.
Primer No. 5'-3 (SEQ ID NO: 7):
   5'-ACGGGATCCAGGACCCTGGCTGGAGAGACA-3'
Primer No. 3'-3 (SEQ ID NO: 8):
   5'-AAGCTCGAGCCGCCGGAACCGCGGCCGGA-3'

The obtained E. *coli* was cultured under shake-flask condition overnight at 37°C in an LB medium containing 5 mL of ampicillin. Then 1,000mL of LB medium was added to the culture and the resulting culture was incubated with shaking for 4 hours at 37°C, followed by addition of 1mL of 500mM isopropyl-1-thio-beta-D-galactoside (IPTG) and incubation with shaking for 3 hours. The resulting culture was centrifuged at 6,000 rpm for 15 minutes at 4°C, and the precipitate was washed twice with phosphate buffered saline (PBS). Next, 20mL of a 1mM EDTA, 20mM Tris-buffered saline (pH7.4) containing 1% Triton X-100 was added to the precipitate, which was then sonicated for 30 seconds for a total of four times to disrupt the cells and to extract the target protein. Then, the suspension was centrifuged at 10,000 rpm for 30 minutes at 4°C and the supernatant was collected. GST - 31kDa secreted Erc/MPF/mesothelin - His protein was purified from the supernatant using Glutathion-sepharose beads (Amersham Biosciences). Then, the GST portion was cleaved and removed by PreScission Protease (Amersham Biosciences) treatment, yielding 31kDa secreted Erc/MPF/mesothelin - His.

### (2) Preparation of Monoclonal Antibody

Mice were immunized by administration of 50 microliter (50 microgram) of the protein obtained by above (1) as an antigen for immunization at one-week or two-week intervals. The antigen for the first immunization was mixed with Freund's complete adjuvant and the antigen for the second or later immunization was mixed with Freund's incomplete adjuvant. Spleen monocytic cells from the immunized mice and the fusion partner X63-Ag8-653 were furnished by polyethylene glycol-mediated cell fusion, and hybridomas were selected by a method described in the literature (J. Immunol. 146:3721-3728). Cells which react with immobilized 31kDa secreted Erc/MPF/mesothelin were selected.

The cells selected in the above were induced to produce antibodies in a serum-free GIT medium (Wako Pure Chemical Industries) until 80% of the cells died. Next, the cells were removed from the medium by centrifugation (1,000rpm, 15min) and ammonium sulfate was added to 50% saturation. The mixture was incubated overnight at 4°C, and then centrifuged (1,000rpm, 30min) to recover the precipitate. The precipitate was then dissolved in a two-fold diluted binding buffer (Protein AMAPS II kit), and IgG was adsorbed onto a protein A column (Pharmacia-Amersham). The antibody was purified by PBS dialysis overnight, thereby giving a plurality of antibodies which recognize 31kDa secreted Erc/MPF/mesothelin. Two types of antibody from these antibodies were named 7E7 and 16K16.

### (3) Confirming the Specificity of Monoclonal Antibodies 7E7 and 16K16 by Western Blotting

The monoclonal antibodies 7E7 and 16K16 obtained in above (2) was confirmed to recognize 31kDa secreted Erc/MPF/mesothelin by Western blotting using samples of Erc/MPF/mesothelin protein, etc. produced by obtained by COS-1 cells or CHO cells forced to express the protein. Prior to the Western blotting, 2-mercaptoethanol (2Me) was added to the samples to reduce. Western blotting was carried out in accordance with a conventional method (e.g., *Bunshi-Seibutsugaku Kiso Jikkenho* [Basic experimental methods in molecular biology] (Nankodo).

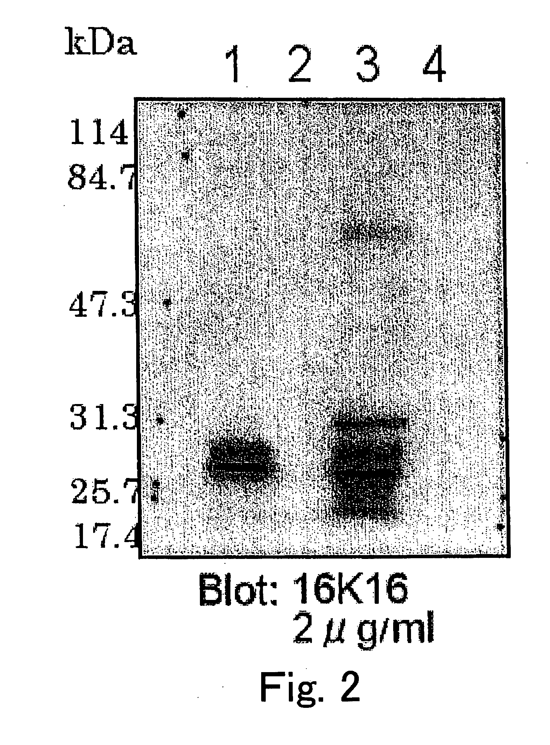

The results of Western blotting are shown in FIGS. 1 and 2. It was confirmed from FIGS. 1 and 2 that both monoclonal antibodies 7E7 and 16K16 showed, bands at the expression sites of 71kDa full-length Erc/MPF/mesothelin and 31kDa secreted Erc/MPF/mesothelin for samples forced to express Erc/MPF/mesothelin. Thus, it was demonstrated that these antibodies have reactivities with respect to Erc/MPF/mesothelin.

### Example 2: Search for Antigen Recognition Sites of Monoclonal Antibodies 7E7 and 16K16

A series of fused proteins which lacked amino acids at the C-terminal region of 31kDa secreted Erc/MPF/mesothelin in six amino acids units were prepared, and reacted with the monoclonal antibodies 7E7 and 16K16 to search for the antigen recognition sites.

First, antisense primers each of which was displaced in 6 amino acids units were prepared based on the template of the GST - 31kDa secreted Erc/MPF/mesothelin - His region produced in Example 1, and used for amplification by a PCR reaction. Next, the products were inserted in pGEX-6P-1 (Amersham Biosciences) so as to give an expression vector, which was then integrated into *E. coli*. The *E. coli* was incubated with shaking overnight at 37°C in 5mL of LB medium containing ampicillin. The cultured E. *coli* was then added to 1,000 mL of LB medium and incubated with shaking for four hours at 37°C. To the incubated culture 1mL of 500mM isopropyl-1-thio-beta-D-galactoside (IPTG) was added and the culture was incubated with shaking for 3hours. The resulting culture was centrifuged at 6,000rpm for 15 minutes at 4°C, and the precipitate was washed twice with a phosphate buffer saline (PBS). Next, 20mL of a 1mM EDTA, 20mM Tris buffered saline (pH7.4) containing 1% Triton X-100 was added. The resulting suspension was sonicated for 30 seconds for a total of four times to disrupt the cells and to extract the target protein. The suspension was then centrifuged and at 10,000rpm for 30 minutes at 4°C, and the supernatant was collected. GST - 31kDa secreted Erc/MPF/mesothelin - His defective proteins which lacked amino acids from 31kDa secreted Erc/MPF/mesothelin in 6 amino acids units were purified from the supernatant using Glutathion-sepharose beads (Amersham Biosciences).

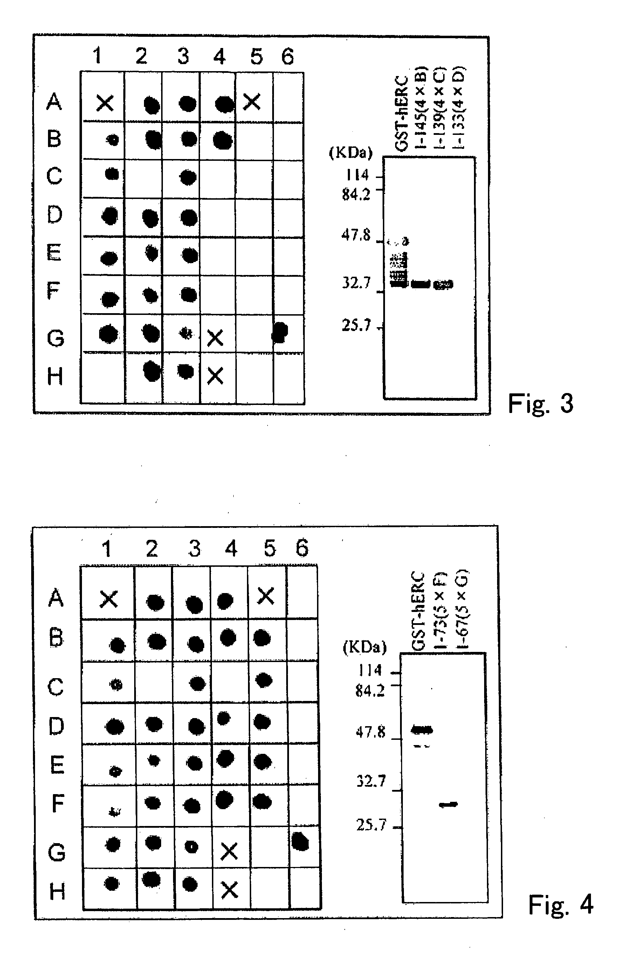

Next, the recognition sites of monoclonal antibodies 7E7 and 16K16 were determined by Western blotting and dot blotting using monoclonal antibodies 7E7 and 16K16 and these defective proteins by employing a conventional method (e.g., *Bunshi-Seibutsugaku Kiso Jikkenho* [Basic experimental methods in molecular biology] (Nankodo).
The results of Western blot and dot blot are shown in FIGS. 3 and 4. Table 1 shows the amino acid sequences of the proteins corresponding to 1 to 6 and A to H in FIGS. 3 and 4. The numbers in the table represent the number of continuous amino acids from the N-terminal amino acid of 31kDa secreted mesothelin to the C-terminal. It was found from these diagrams that monoclonal antibody 7E7 recognizes the region of amino acid 134 to 139 from the N-terminal, and monoclonal antibody 16K16 recognizes the region of amino acid 68 to 73 from the N-terminal.

**Table 1**

| | 1 | 2 | 3 | 4 | 5 | 6 |
|---|---|---|---|---|---|---|
| A | GST-hERC | 247 | 199 | 151 | 103 | 55 |
| B | 289 | 241 | 193 | 145 | 97 | 49 |
| C | 283 | 235 | 187 | 139 | 91 | 43 |
| D | 277 | 229 | 181 | 133 | 85 | GST |
| E | 271 | 223 | 175 | 127 | 79 | -mRNA |
| F | 265 | 217 | 169 | 121 | 73 | -mRNA |
| G | 259 | 211 | 163 | 115 | 67 | GST-hERC |
| H | 253 | 205 | 157 | 109 | 61 | GST |

### Example 3: Production of Polyclonal Antibodies (1)

### (1) Production of Antigen for immunization

A peptide having amino acid sequence (d) below which corresponds to an internal sequence of human 31kDa secreted Erc/MPF/mesothelin was purchased as a HPLC chromatography-purified preparation from Itoham Foods, Inc. This peptide was used as the antigen for producing the polyclonal antibodies.
(d) CPGPLDQDQQEAARAALQG (SEQ ID NO: 5; the sequence of amino acids 211 to 229 in SEQ ID NO: 1)

### (2) Production of Polyclonal Antibodies

First, 100 microgram of the peptide produced in above (1) was mixed with an equal amount of Freund's complete adjuvant to give an emulsion, which was then used to immunize rabbits. One week after immunization, 100 microgram of the antigen was mixed with an equal amount of Freund's incomplete adjuvant to give an emulsion, which was then used for booster immunization to the rabbits. The same booster immunization was subsequently applied three times at one-week intervals. The rise in titer for the antigen was then confirmed by the ELISA method using immobilized antigen peptide, and the entire blood of the animal was collected and centrifuged at 1,500 rpm for 15 minutes to separate off the antiserum. Polyclonal antibodies were obtained by Antigen-specific purification using an affinity column to which antigen peptide had been bonded, and were named antibodies 211.

### Example 4: Production of Polyclonal Antibodies (2)

### (1) Production of Antigen for immunization

A peptide having an amino acid sequence consisted of following amino acid sequence (a) which corresponds to an internal sequence of human 31kDa secreted Erc/MPF/mesothelin and cysteine (c) attached to C-terminal of the amino acid sequence (a) was purchased in a HPLC chromatography-purified preparation from Itoham Foods, Inc. The peptide was used as the antigen for producing polyclonal antibodies.
(a) SRTLAGETGQEAAPLDGVC (SEQ ID NO: 2; the sequence of amino acids 34 to 51 in SEQ ID NO: 1)

### (2) Production of Polyclonal Antibody

First, 100 microgram of the peptide produced in above (1) was mixed with an equal amount of Freund's complete adjuvant to give an emulsion, which was then used to immunize rabbits. One week after immunization, 100 microgram of the antigen was mixed with an equal amount of Freund's incomplete adjuvant to give an emulsion, which was then used for booster immunization to the rabbits. The same booster immunization was subsequently applied three times at one-week intervals. The rise in titer for the antigen was then confirmed by the ELISA method using immobilized antigen peptide, and the entire blood of the animal was collected and centrifuged at 1,500 rpm for 15 minutes to separate off the antiserum. Polyclonal antibodies were obtained by Antigen-specific purification using an affinity column to which antigen peptide had been bonded, and was named antibodies 34.

### Reference Example 1: Production of Polyclonal Antibodies

### (1) Production of Antigen for immunization

A peptide having an amino acid sequence consisted of following amino acid sequence (e), which corresponds to an internal sequence of human 31kDa secreted Erc/MPF/mesothelin and cysteine (c) attached to C-terminal of the amino acid sequence (e) was purchased in a HPLC chromatography-purified preparation from Auspep Corporation. The peptide was used as the antigen for producing polyclonal antibodies.
(e) RQPERTILRPRFRR (SEQ ID NO: 9; the sequence of amino acids 282 to 295 in SEQ ID NO: 1)

### (2) Production of Polyclonal Antibodies

First, 100 microgram of the peptide produced in above (1) was mixed with an equal amount of Freund's complete adjuvant to give an emulsion, which was then used to immunize rabbits. One week after immunization, 100 microgram of the antigen was mixed with an equal amount of Freund's incomplete adjuvant to give an emulsion, which was then used for booster immunization to the rabbits. The same booster immunization was subsequently applied three times at one-week intervals. The rise in titer for the antigen was then confirmed by the ELISA method using immobilized antigen peptide, and the entire blood of the animal was collected and centrifuged at 1,500 rpm for 15 minutes to separate off the antiserum. Polyclonal antibodies were obtained by Antigen-specific purification using an affinity column to which antigen peptide had been bonded, and was named antibody 4.

### (3) Confirming the Specificity of Polyclonal Antibodies 4 by Western Blotting

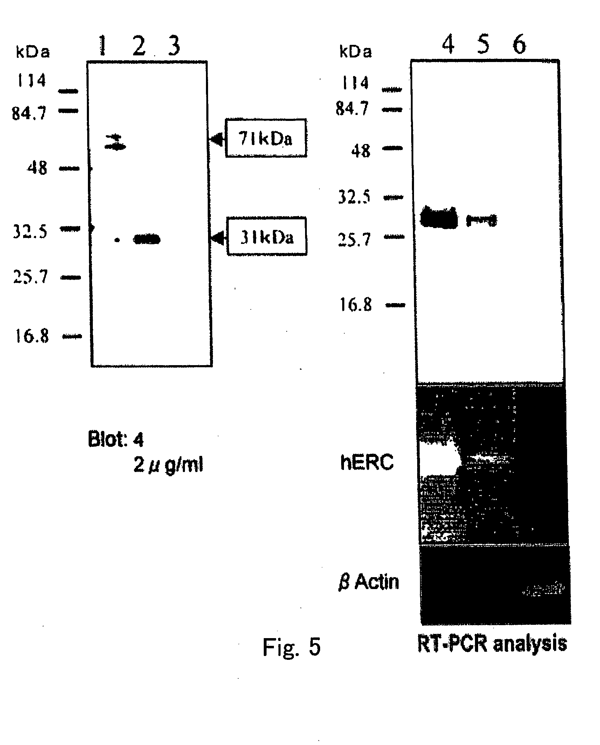

Next, polyclonal antibodies 4 obtained in above (2) was confirmed to recognize 31kDa Erc/MPF/mesothelin, by Western blotting, following a conventional method as described above with Erc/MPF/mesothelin protein produced by COS-1 cells which were forced to express the protein. The results of Western blotting are shown in FIG. 5. The samples were the same as those used in Example 1(3). From the results in FIG. 5, the polyclonal antibodies 4 were confirmed to have a reactivity with samples from cells forced to express Erc/MPF/mesothelin.

### Example 5: Assaying 31kDa Secreted Erc/MPF/Mesothelin in pleural effusion of Mesothelioma Patients

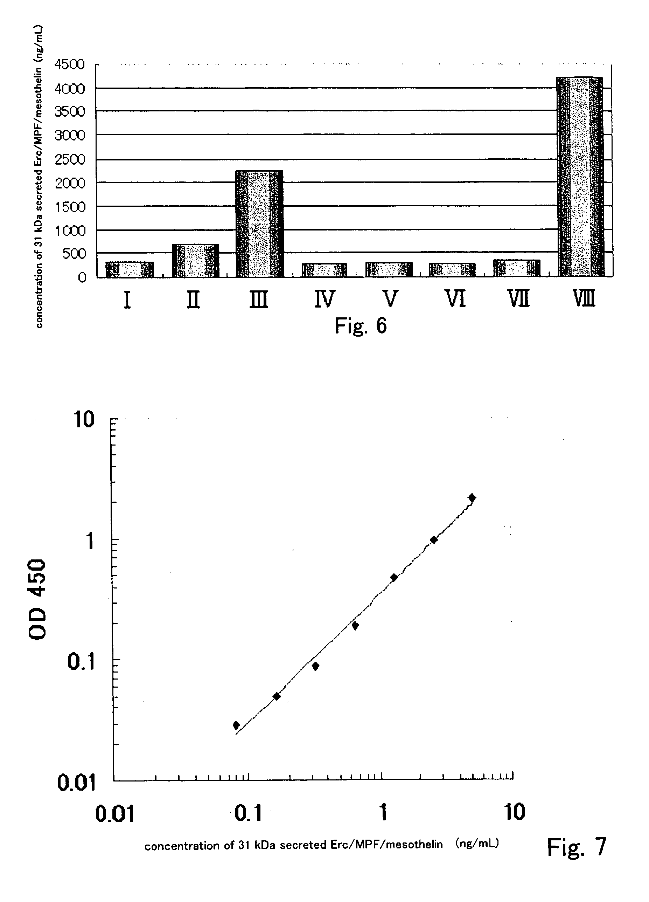

An ELISA system was constructed according to the methods described in Example 6 and Reference Example 2 by using combination of Monoclonal antibodies or polyclonal antibodies prepared in Examples 1, 3 and 4 and Reference Example 1. with this ELISA system, the concentration of 31kDa secreted Erc/MPF/mesothelin in the pleural effusion of mesothelioma patients was measured. The results are shown in FIG. 6.

From FIG. 6, the measured concentrations of 31kDa secreted Erc/MPF/mesothelin in pleural effusion were higher in the following order: combination of monoclonal antibodies 7E7 and 16K16, combination of monoclonal antibody 7E7 and polyclonal antibodies 34, combination of monoclonal antibody 211 and polyclonal antibody 34. In these results, the concentration measured by the systems which use antibody 4 was low, which indicated that there were few 31kDa secreted Erc/MPF/mesothelin molecules retaining the amino acids to the C-terminal in the pleural effusion. On the other hand, , the concentration measured by the systems which use antibody 34 were lower in the order antibody 211, antibody 7E7, which indicate that 31kDa secreted Erc/MPF/mesothelin molecules retaining the N-terminal had been decomposed to lack the region from the C-terminal to the site recognized by the antibody 7E7.

### Example 6: Production of ELISA Assay System (7E7 - 16K16 Kit)

### (1) Production of Standard Substance

The concentration of 31kDa secreted Erc/MPF/mesothelin expressed in COS-1 cells was measured based on the antigen protein (31kDa secreted Erc/MPF/mesothelin - His) used in antibody production, and used as the standard substance for the ELISA assay system.

### (2) Preparing a Conjugate of Monoclonal Antibody 16K16 and HRP

A conjugate of monoclonal antibody 16K16 obtained in Example 1 and HRP was prepared as follows. The required amount of HRP was dissolved in distilled water and oxidized with NaIO₄, and then dialyzed overnight against 1mM acetate buffer solution (pH 4.4). In addition, 1 to 10 mg of monoclonal antibody 16K16 was also dialyzed overnight against 0.1 M carbonate buffer solution (pH 9.5). The dialyzed antibody 16K16 and the dialyzed HRP were mixed at the rate of 0.4mg HRP per 1mg of antibody and reacted at room temperature for 2 hours. Next, NaBH₄ was added thereto and reacted in ice for 2 hours, and then the reaction product was dialyzed overnight against PBS. Then, the reaction product was gel filtered to prepare creating a conjugate of monoclonal antibody 16K16 and HRP.

### (3) Preparation of ELISA Assay System

A sandwich ELISA system was constructed as follows. First, 100 microliter of 10 microgram/mL antibody 7E7 was added to each well of a 96-well ELISA plate and reacted overnight at 4°C. Then, 1% BSA/PBS/NaN₃ solution was added to block, and the plate was used as a plate for sandwich ELISA assays. The conjugate of monoclonal antibody 16K16 and HRP prepared in above (2) was used as the standard antibody.

Suitably diluted standard substance and sample were added to each well and reacted at 37°C for 1 hour (primary reaction), and the wells were rinsed. Then, horseradish peroxidase (HRP)-labeled monoclonal antibody 16K16 was added and reacted at 4°C for 30 minutes (secondary reaction) the wells were rinsed. Next, a tetramethyl benzidine (TMB)-containing substrate solution was added, and the mixture was incubated at room temperature for 30 minutes. After the incubation, a reaction stop solution (1N H₂SO₄) was added to stop the reaction. The absorbance at a wavelength of 450 nm was then measured, and the concentration of 31kDa secreted Erc/MPF/mesothelin in the sample was calculated by a standard curve prepared from the standard substance.

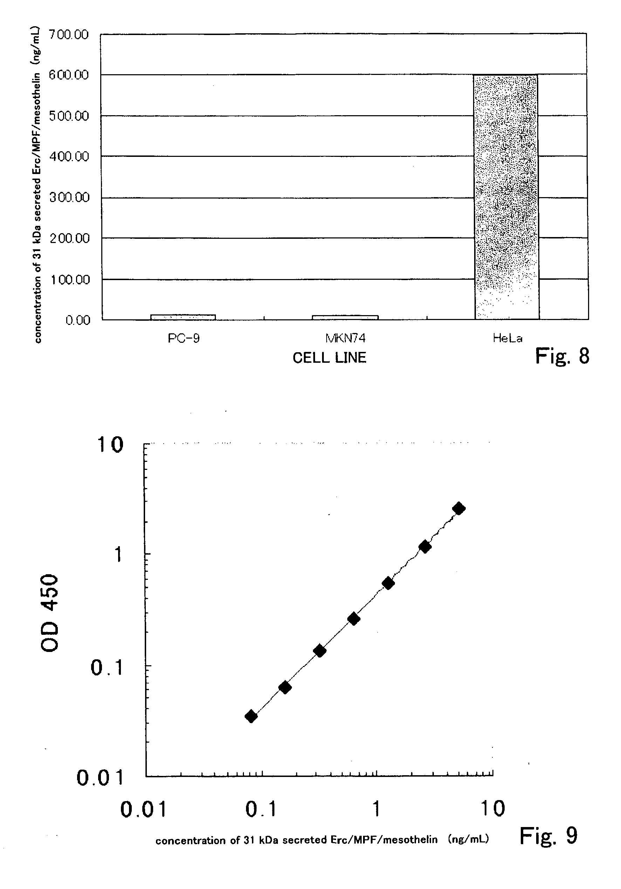

FIG. 7 shows an example of a typical standard curve. FIG. 8 shows concentrations of the 31kDa secreted Erc/MPF/mesothelin in the culture supernatants of various cultured cells measured by the assay system. From these results, it was found that a large amount of 31kDa secreted Erc/MPF/mesothelin is produced in HeLa cells.

### Reference Example 2: Preparation of ELISA Assay Systems (7E7 - 4 Kits)

### (1) Preparing a Conjugate of Polyclonal Antibodies 4 and HRP

A conjugate of the polyclonal antibodies 4 obtained in Reference Example 1 and HRP was prepared in accordance with Example 6 (2).

### (2) Preparation of ELISA Assay System

A sandwich ELISA system using monoclonal antibody 7E7 obtained in Example 1 and polyclonal antibodies 4 obtained in Reference Example 1 was constructed as follows. First, 100 microliter of 10 microgram/mL of monoclonal antibody 7E7 was added to each well of a 96-well ELISA plate and reacted overnight at 4°C. Then, 1% BSA/PBS/NaN₃ solution was added to block and the plate was used as a plate for sandwich ELISA assays. The conjugate of polyclonal antibody 4 and HRP prepared in above (1) was used as the standard antibody.

Suitably diluted standard substance and sample was added to each well and reacted at 37°C for 1 hour (primary reaction), and the wells were rinsed. Then, horseradish peroxidase (HRP)-labeled antibody 4 was added and reacted at 4°C for 30 minutes (secondary reaction) and the wells were rinsed. Next, a tetramethyl benzidine (TMB)-containing substrate solution was added, and the mixture was incubated at room temperature for 30 minutes. After the incubaton, a stop solution (1N H₂SO₄) was added to stop the reaction. The absorbance at a wavelength of 450 nm was then measured, and the concentration of 31kDa secreted Erc/MPF/mesothelin in the sample was calculated by a standard curve prepared from the standard substance.

FIG. 9 shows an example of a typical standard curve. FIG. 10 shows concentrations of the 31kDa secreted Erc/MPF/mesothelin in the culture supernatants of various cultured cells measured by the assay system. In addition, FIG. 11 shows concentrations of the 31kDa secreted Erc/MPF/mesothelin in the sera of healthy subjects. The average value in the serum of healthy subjects was 7.66 ng/mL, the average value in heparin added plasma was 10.60 ng/mL, and the average value in EDTA added plasma was 11.77 ng/mL.

### Example 7: Assaying the Sera of Various Types of Patients

Using the ELISA assay system (7E7 - 16K16 kit) prepared in Example 6, the 31kDa secreted Erc/MPF/mesothelin concentrations in the sera of 27 mesothelioma patients, 28 pleural thickening patients, 6 patients with benign pleurisy and diffuse pleural thickening, 11 pulmonary fibrosis patients who was exposed to asbestos, and 8 patients with other diseases to be distinguished from mesothelioma (including lung cancer) were measured as follows. For the comparison, the samples were assayed in similar method using the ELISA assay system (7E7 - 4 kit) prepared in Reference Example 2.

The serum samples were diluted 8-fold with PBS shortly after collection, and used for measurement according to the method described in Example 3. Concentrations of the 31kDa secreted Erc/MPF/mesothelin were determined from the absorbances of the diluted samples, and the determined concentrations were converted to the concentrations of the original samples (8-fold). The results of these assays are shown in Table 2.

**[Table 2]**

| Diseases | Mesothelioma (all) | | Pleural thickening | | Benign pleurisy/ diffuse pleural thickening | | Exposure to asbestos/ pulmonary fibrosis | | Other diseases to be distinguished (including lung cancer) | |
|---|---|---|---|---|---|---|---|---|---|---|
| Type of kit* | A | B | A | B | A | B | A | B | A | B |
| Measured values (ng/mL) | 1.08 | 0.62 | 0.74 | 6.74 | 1.18 | 0.30 | 1.97 | 0.58 | 1.31 | 0.48 |
| | 1.06 | 0.80 | 0.93 | 0.74 | 1.80 | 0.75 | 1.99 | 0.03 | 1.69 | 0.74 |
| | 1.64 | 1.12 | 1.32 | 0.24 | 2.52 | 1.97 | 2.80 | 1.22 | 1.95 | 0.71 |
| | 1.95 | 1.24 | 1.68 | 1.26 | 2.55 | 0.57 | 3.07 | 2.10 | 3.53 | 6.57 |
| | 2.60 | 1.18 | 2.15 | 1.36 | 3.41 | 1.10 | 3.23 | 1.08 | 3.77 | 0.68 |
| | 2.98 | 1.04 | 2.16 | 1.10 | 4.00 | 1.98 | 3.26 | 4.13 | 4.25 | 0.99 |
| | 3.08 | 0.85 | 2.30 | 1.04 | -- | -- | 3.64 | 1.96 | 6.03 | 4.33 |
| | 3.29 | 1.62 | 2.42 | 2.25 | -- | -- | 3.83 | 1.00 | 9.76 | 1.88 |
| | 4.26 | 1.75 | 2.64 | 0.43 | -- | -- | 4.42 | 1.73 | -- | -- |
| | 5.81 | 2.85 | 2.72 | 1.07 | -- | -- | 4.48 | 3.15 | -- | -- |
| | 5.91 | 2.89 | 2.86 | 2.02 | -- | -- | 5.27 | 7.66 | -- | -- |
| | 6.15 | 3.71 | 3.06 | 1.34 | -- | -- | -- | -- | -- | -- |
| | 6.44 | 2.70 | 3.14 | 4.34 | -- | -- | -- | -- | -- | -- |
| | 6.45 | 4.79 | 3.37 | 0.99 | -- | -- | -- | -- | -- | -- |
| | 6.60 | 1.92 | 3.57 | 2.36 | -- | -- | -- | -- | -- | -- |
| | 11.10 | 5.64 | 3.59 | 2.07 | -- | -- | -- | -- | -- | -- |
| | 11.70 | 4.03 | 3.66 | 1.22 | -- | -- | -- | -- | -- | -- |
| | 12.86 | 5.87 | 3.70 | 1.15 | -- | -- | -- | -- | -- | -- |
| | 13.47 | 21.46 | 3.82 | 1.16 | -- | -- | -- | -- | -- | -- |
| | 17.40 | 15.90 | 4.04 | 2.35 | -- | -- | -- | -- | -- | -- |
| | 24.08 | 6.35 | 4.16 | 1.16 | -- | -- | -- | -- | -- | -- |
| | 27.58 | 10.32 | 4.53 | 2.25 | -- | -- | -- | -- | -- | -- |
| | 35.87 | 25.64 | 4.67 | 2.24 | -- | -- | -- | -- | -- | -- |
| | 45.44 | 14.68 | 5.25 | 3.04 | -- | -- | -- | -- | -- | -- |
| | 45.49 | 48.18 | 5.68 | 2.51 | -- | -- | -- | -- | -- | -- |
| | 50.90 | 53.13 | 5.76 | 2.18 | -- | -- | -- | -- | -- | -- |
| | 55.59 | 24.17 | 5.86 | 1.68 | -- | -- | -- | -- | -- | -- |
| | -- | -- | 7.45 | 6.82 | -- | -- | -- | -- | -- | -- |
| Cut-off value | 6 | 3 | -- | -- | -- | -- | -- | -- | -- | -- |
| Positive cases | 16/27 | 14/27 | 1/28 | 4/28 | 0/6 | 0/6 | 0/11 | 3/11 | 2/8 | 2/8 |

| | | | | | | | | | | |
|---|---|---|---|---|---|---|---|---|---|---|
| * Type of Kit: A: 7E7 - 16K16 kit B: 7E7 - 4 kit | | | | | | | | | | |

These results indicate that the concentration of a 31kDa secreted Erc/MPF/mesothelin in mesothelioma patients was ranged from 1.06 to 55.59ng/mL with the average value of 15.21ng/mL; that the concentration of a 31kDa secreted Erc/MPF/mesothelin in patients with pleural thickening was ranged from 0.74 to 7.45ng/mL with the average value of 3.47ng/mL; that the concentration of a 31kDa secreted Erc/MPF/mesothelin in patients with benign pleurisy and diffuse pleural thickening was ranged from 1.18 to 4.00ng/mL with the average being 2.58ng/mL; that the concentration of a 31kDa secreted Erc/MPF/mesothelin in individuals who were exposed to asbestos and in pulmonary fibrosis was ranged from 1.97 to 5.27ng/mL with the average value of 3.45ng/mL; and that the concentration of a 31kDa secreted Erc/MPF/mesothelin in other diseases to be distinguished from mesothelioma (including lung cancer) was ranged from 1.31 to 9.76ng/mL with the average value of 4.04ng/mL. It was found that the concentration of 31kDa secreted Erc/MPF/mesothelin was higher in the sera of mesothelioma patients than patients with other diseases.

By setting the cut-off value as 6.0ng/mL in the 7E7 - 16K16 kit and setting the cut-off value as 3.0ng/mL in the 7E7 - 4 kit, the positive ratio shown in the Table 2 was obtained. In the result measured by the 7E7 - 4 kit, the positive ration was low in mesothelioma patients, and high in subjects of pleural thickening and exposure to asbestos/pulmonary fibrosis. In other diseases to be distinguished from mesothelioma, both kits showed the same positive ratio. From overall results, the 7E7 - 16K16 kit was able to diagnose mesothelioma patients at a higher sensitivity and a higher specificity.

### Example 8: Assays of the Sera and Plasma of Healthy Subjects

Using the ELISA assay system (7E7 - 16K16 kit) prepared in Example 6, the 31kDa secreted Erc/MPF/mesothelin concentration in the sera and EDTA plasma of 52 healthy subjects was measured as follows.

The serum samples were diluted 8-fold with PBS shortly after collection, and used for measurement according to the method described in Example 3. Concentrations of the 31kDa secreted Erc/MPF/mesothelin were determined from the absorbances of the diluted samples, and the determined concentrations were converted to the concentrations of the original samples (8-fold). The results of these assays are shown in Table 3.

**Table 3**

| | 31kDa secreted Erc/MPF/mesothelin concentration (ng/mL) | |
|---|---|---|
| | Serum | Plasma |
| Average | 3.36 | 3.60 |
| Standard deviation | 1.72 | 1.70 |
| Lowest value | 1.03 | 1.44 |
| Highest value | 8.19 | 8.29 |

These results indicate that the concentration of a 31kDa secreted Erc/MPF/mesothelin in the sera of healthy subjects was ranged from 1.03 to 8.19ng/mL with the average value of 3.36ng/mL, and that the concentration of a 31kDa secreted Erc/MPF/mesothelin in the plasma of healthy volunteers was ranged from 1.44 to 8.29ng/mL with the average value of 3.60ng/mL.

### Example 9: Confirmation of the Stability of 31kDa Secreted Erc/MPF/mesothelin in Serum and Plasma

Using the ELISA assay system (7E7 - 16K16 kit) prepared in Example 6, the stability of 31kDa secreted Erc/MPF/mesothelin in the sera and EDTA plasma of five healthy subjects (Subjects a to e) was measured as described below.

Blood samples collected from the subjects were divided up into a number of vacuum blood collection tubes, then incubated at room temperature for one hour and centrifuged at 1,500 rpm for 10 minutes to separate the serum, which was then immediately frozen and stored at -20°C. These samples were used as controls. In addition, the other sample was prepared by leaving blood for one hour at room temperature after collection, then additionally leaving the blood for 16 hours at room temperature, then centrifuging the blood to separate off the serum, and freezing the serum (Test Group A). The other sample was prepared by leaving blood for one hour at room temperature, then centrifuging the blood to separate off the serum, then additionally leaving the serum for 16 hours at room temperature, and freezing the serum (Test Group B). The frozen samples were thawed and diluted 8-fold with PBS, and assayed in accordance with the methods described in the above examples. The concentrations of the 31kDa secreted Erc/MPF/ mesothelin were then determined from the absorbance of the diluted samples, and these concentration were converted to the concentration in the original samples (8-fold). FIG. 12 shows the results of assays using the 7E7 - 16K16 kit. For the comparison, the samples were assayed in similar method using the assay system prepared in Reference Example 2 (7E7 - 4 kit). The results are shown in FIG. 13.

As a result, the concentrations of Erc/MPF/mesothelin did not significantly differ between the samples prepared by leaving blood for 16 hours at room temperature after collection and the samples prepared by leaving serum for 16 hours at room temperature after separation following to blood collection, and were stable under these experimental conditions. This result means that the 7E7 - 16K16 kit is a suitable assay system for accurate measurement. Of the five healthy subjects, the results of Subject b in Test Group B were excluded because the data for the sample prepared by leaving for 16 hours could not be obtained.

In a similar experiment with the 7E7 - 4 kit using an antibody which recognizes a C-terminal region of 31kDa secreted Erc/MPF/mesothelin as a detection antibody, the sample of blood serum was incubated at room temperature for 3 hours after collection of blood and prior to separation showed the measured value that was 5 to 30% lower than a control. As for the sample of serum prepared by leaving at room temperature after blood collection and separation until it was frozen, in the case of at 3 hours incubation, the sample showed the measured value that was 5 to 30% lower. These results show that when the blood or the serum separated from the blood was incubated at room temperature for 3 hours or longer, fragmentation of the 31kDa secreted Erc/MPF/mesothelin may occur.

### Example 10: Detection of the Presence of Various Secreted Erc/MPF/Mesothelin Fragments in the Pleural effusion of Mesothelioma Patients

In Example 9, it was shown that 31kDa secreted Erc/MPF/mesothelin may decompose and fragment in serum. Specifically, during the period of time (3 hours or more) that the sample was kept after blood collection and serum separation, C-terminal regions of the 31kDa secreted Erc/MPF/mesothelin decomposed and were lost, making it impossible to measure with assay kits that employ antibodies which recognize said regions. As a result, measured values emerge which would seem to indicate that the target protein (31kDa secreted Erc/MPF/mesothelin) is not present. However, the molecules prior to decomposition should have been present within the blood, and such tests possibly give erroneous results. It is thus very important to use antibodies having appropriate recognition sites. To confirm this, Western blotting was carried out by the method described below with monoclonal antibody 7E7 and polyclonal antibodies 34, 211 and 4 prepared in the above examples and reference examples. The samples used for the Western Blotting were pleural effusions from mesothelioma patient that had been left at room temperature following collection which were thought to be comparable to the sample which is incubated at room temperature for 3 hours as blood or separated serum.

First, the monoclonal antibody 7E7 and polyclonal antibodies 34 and 4 were respectively mixed and bonded with Formyl-Cellulofine (Seikagaku Corporation) using a reducing agent, and the resulting conjugate was packed into a column. Next, the pleural effusion from a mesothelioma patient was fractionated by this column with a strongly acidic solution, a high-salt concentration solution, etc. Specifically, the pleural effusion from the mesothelioma patient was passed through a column in which polyclonal antibody 4 had been bonded to separate into an adsorbed portion (C-terminal antibody column adsorbed fraction) and a non-adsorbed portion. Next, the portion that did not adsorb to the column was passed through a column in which polyclonal antibody 34 had been bonded to separate into an adsorbed portion and a non-adsorbed portion (N-terminal, C-terminal antibody column non-adsorbed fraction). In addition, the portion that adsorbed to the column was passed through a column in which monoclonal antibody 7E7 had been bonded, thereby separating it into an adsorbed portion (antibody 7E7 column adsorbed fraction) and a non-adsorbed portion (antibody 7E7 column non-adsorbed fraction). Using these respective fractions as samples, Western blotting with monoclonal antibody 7E7 and polyclonal antibodies 34, 211 and 4 was carried out. The results are shown in Fig. 14.

From the results, it became apparent that a plurality of fragments (N-terminal side fragments) which lacked C-terminal regions of the 31kDa secreted Erc/MPF/mesothelin were present in the sample of pleural effusion from mesothelioma patients which had been kept at room temperature following collection. The results also demonstrated that a plurality of N-terminal side fragments were present when blood or serum separated from the blood had been kept at room temperature for 3 hours or more.

In these examples, some fragments could not be detected due to differences in the recognition sites of the antibodies used. Thus, in order to detect at a high efficiency the 31kDa secreted Erc/MPF/mesothelin that was originally present in the body, it is important to select suitable antibodies by searches such as above described. By the search, it is able to construct an assay system for detecting a target protein at a high sensitivity. These assay systems which enables detection at a high sensitivity provide the detection of the presence even at smaller amounts and early detection and diagnosis.

### Example 11: Post-Surgical Monitoring

Blood was collected pre- and post-operatively from a patient who had been diagnosed with malignant mesothelioma and given surgical operation, the serum was separated from the blood, and the concentration of 31kDa secreted Erc/MPF/mesothelin in the serum was measured using both the ELISA assay system prepared in Example 6 (7E7 - 16K16 kit) and the ELISA assay system prepared in Reference Example 2 (7E7 - 4 kit).

As a result, the concentration measured by 7E7 - 16K16 kit before the operation was 35.87ng/mL, which was dropped to 3.21ng/mL after the operation. The concentration measured by the 7E7 - 4 kit before the operation was 25.64ng/mL, which was dropped to 1.47ng/mL after the operation. This demonstrated that, by assaying the 31kDa secreted Erc/MPF/mesothelin concentration in the serum both pre- and post-operatively, it is possible to confirm the removal of mesothelioma cells by surgery. Conversely, because these results imply that 31kDa secreted Erc/MPF/mesothelin in blood serum arises from the presence of mesothelioma cells, it is possible to predict relapses by monitoring the concentration of 31kDa secreted Erc/MPF/mesothelin following surgery.

### Example 12: Measuring the Sera of Various Types of Patients

The concentration of 31kDa secreted Erc/MPF/mesothelin was measured in the same way as in Example 7 in the sera of 39 mesothelioma patients, 102 healthy subjects, 201 patients with asbestos-related diseases/individuals who were exposed to asbestos (98 pleural plaque patients, 83 individuals who were exposed to asbestos, 6 patients with asbestosis, 14 patients with benign asbestos pleurisy), 45 lung cancer patients and 8 patients with other diseases to be distinguished from mesothelioma.

The results are shown in FIG. 15. As shown in the figure, the Erc/MPF/mesothelin concentration assayed using the antibody of the invention indicated that the values obtained in mesothelioma patients were higher than the values obtained in patients with lung diseases other than mesothelioma, individuals who was exposed to asbestos and healthy subjects. This demonstrated that the antibody of the present invention can be used in the effective diagnosis of mesothelioma.

The entire contents of Japanese Patent Application No. 2006-331409 filed in Japan on December 8, 2006, which serves as the basis for the priority claim of the present invention, are incorporated herein by reference. Also, the entire contents of all patents, patent applications and reference documents cited in this application are incorporated herein by reference.

## Claims

1. An antibody which recognizes a peptide which lacks amino acid(s) from the C-terminal of 31kDa secreted Erc/MPF/mesothelin.

2. An antibody which recognizes an N-terminal side fragment of fragments formed by leaving 31kDa secreted Erc/MPF/mesothelin in a bodily fluid at room temperature.

3. An antibody which recognizes an N-terminal side fragment of fragments formed by leaving 31kDa secreted Erc/MPF/mesothelin in blood or serum at room temperature.

4. An antibody which recognizes an N-terminal side fragment of fragments formed by leaving 31kDa secreted Erc/MPF/mesothelin in blood or serum at room temperature for at least three hours.

5. An antibody having a recognition site which includes amino acids 34 to 229 of the amino acid sequence of SEQ ID NO: 1.

6. An antibody having a recognition site which includes amino acids 34 to 139 of the amino acid sequence of SEQ ID NO: 1.

7. An antibody having a recognition site which includes amino acids 68 to 139 of the amino acid sequence of SEQ ID NO: 1.

8. The antibody of any one of claims 1 to 7, which further recognizes 31kDa secreted Erc/MPF/mesothelin.

9. A diagnostic agent for mesothelioma, comprising the antibody of any one of claims 1 to 8.

10. A diagnostic kit for mesothelioma, comprising the antibody of any one of claims 1 to 8.

11. A diagnostic kit for mesothelioma, comprising, a combination of a first reagent containing a first antibody that recognizes a peptide which lacks amino acid(s) from the C-terminus of 31kDa secreted Erc/MPF/mesothelin, and a second reagent containing a second antibody that recognizes a peptide which lacks amino acid(s) from the C-terminus of 31kDa secreted Erc/MPF/mesothelin, wherein the second antibody recognize a site which is different from the first antibody.

12. A method of diagnosing mesothelioma, the method comprising: assaying an amount of 31kDa secreted Erc/MPF/mesothelin in a sample with the diagnostic kit for mesothelioma according to claim 10 or 11, and using the assayed amount as an indicator.

13. The method of diagnosing mesothelioma according to claim 12, wherein the sample is serum, plasma, a pleural effusion or ascites.
